# EUROPEAN PATENT SPECIFICATION

(11) **EP 1 233 941 B1**
(45) Date of publication and mention of the grant of the patent: **24.05.2006**
(21) Application number: 00905430.5
(22) Date of filing: 17.02.2000
(51) Int. Cl.: C07C 209/36, C07C 211/56, C07C 209/00

(54) **THE PREPARATION OF 4,4'-DINITRODIPHENYLAMINE FROM UREA AND NITROBENZENE**
DIE HERSTELLUNG VON 4,4'-DINITROPHENYLAMIN AUS HARNSTOFF UND NITROBENZOL
PREPARATION DE 4,4'-DINITRODIPHENYLAMINE A PARTIR D'UREE ET DE NITROBENZENE

(30) Priority: 03.12.1999 KR 9954650
(43) Date of publication of application: 28.08.2002
(73) Proprietor: Korea Kumho Petrochemical Co. Ltd., Chongno-ku, Seoul 110-110 (KR)
(72) Inventor: JOO, Young J., Yuseong-gu, Taejeon 305-345 (KR); KIM, Jin-Eok, Yuseong-gu, Taejeon 305-345 (KR); WON, Jeong-Im, Yuseong-gu, Taejeon 305-345 (KR); HWANG, Kum-Ui, Yuseong-gu, Taejeon 305-345 (KR)
(74) Representative: Kador & Partner
(86) International application number: PCT/KR2000/000129
(87) International publication number: WO 2001/040161

(56) References cited:
- WO-A-94/08953
- JP-A- 63 316 762
- US-A- 4 187 248
- US-A- 4 990 673
- DATABASE CAPLUS [Online] CHEMICAL ABSTRACTS SERVICE, COLUMBUS, OHIO, US; 1976 IIDA H ET AL.: "Studies on the base-catalysed reaction.VVI. The reactions of p-nitroaniline with nitrobenzene derivatives in strongly basic solutions" Database accession no. 1976:134795 XP002235448 & NIPPON KAGAKU KAISHI, no. 1, 1976, pages 138-143,

## Description

### Technical Field

The present invention relates in general to a method of preparing 4,4'-dinitrodiphenylamine by reacting urea and nitrobenzene in a polar organic solvent in the presence of an organic or inorganic base. More particularly, the present invention relates to a method of selectively preparing 4,4'-dinitrodiphenylamine by dissolving urea and nitrobenzene in a polar organic solvent, and then by allowing those urea and nitrobenzene to react together in the resulting solution while using an inorganic base, such as sodium hydroxide, or an organic base, such as tetramethylammonium hydroxide (hereinafter, called "TMA(OH)").

### Background Art

As 4,4'-dinitrodiphenylamine is easily reduced into 4,4'-diaminodiphenylamine, it is mainly used as a raw material for an antioxidant for a dye or rubber. Japanese Patent Publication No. Heisei 10-168038 (1998) describes a method for synthesizing diaminodiphenylamine synthesizing method involving a reduction of dinitrodiphenylamine. In the above mentioned patent publication, a diaminodiphenylamine compound is described as being greatly effective for an intermediate of an antioxidant or antiaging agent. 4,4'-diaminodiphenylamine or its derivatives, besides being used as an intermediate of an additive to rubber, are also used as an intermediate of dyes, agricultural chemicals or medical substances.

An example of conventional methods for preparing 4,4'-dinitrodiphenylamine involves nitration and deacetylation of N-acetyldiphenylamine. Such a method, however, is problematic in that the nitration is non-uniformly carried out. Furthermore, there is an inconvenience in that nitrodiphenylamine inevitably produced should be removed using a recrystalization from alcohol. With another method for preparing 4,4'-dinitrodiphenylamine, US Patent No. 4,990,673 (1991) discloses a method in which 4-chloroaniline is reacted with alkali metal cyanate to produce 4,4'-dinitrodiphenylamine. However, a disadvantage with the latter method is that the reaction must be carried out at temperature of 160 °C or above for an extended period of time of more than 15 hours.

In addition, known is another method of preparing dinitrodiphenylamine derivative by a reaction of 4-nitroaniline with a nitrobenzene derivative (see, J. Am. Chem. Soc., 1976, (1), pp 138-143). Such a reference describes a method in which 4-nitroaniline and nitrobenzene derivatives are allowed to react using potassium t-butoxide (t-BuOK) as a base, thereby producing 2,4'-dinitrodiphenylamine or 4,4'-dinitrodiphenylamine depending on the amount of the base, or alternatively producing 5-chloro (bromo)-2,9'-dinitrodiphenylamine using halogen as a substitute.

The present invention relates to a method of selectively preparing 4,4'-dinitrodiphenylamine using a nucleophilic aromatic substitution for hydrogen (hereinafter, called "NASH"). The NASH reaction recently proposed is advantageous in that, since amine or amide is reacted directly with nitrobenzene or its derivative in the presence of a base, any hazardous material or any intermediate, difficult to be removed, is not produced.

A process is also known wherein aniline and nitrobenzene are directly reacted in the presence of a base, such as "TMA(OH)", to prepare 4-nitrodiphenylamine (hereinafter, called "4-NDPA") and 4-nitrosodiphenylamine. See, J. Am. Chem. Soc., 1992, 114(23), 9237-8; US Patent No. 5,117,063; US Patent No.5,253,737; US Patent No. 5, 331, 099; US Patent No.5,453,541; US Patent No. 5,552,531; and US Patent No 5,633,407.

In addition, the use of the NASH reaction is described in US Patent No. 5,436,371, US Patent No. 5,117,063, and International Patent Publication No. WO 93/24447. These patents disclose a method of synthesizing N-(4-nitrophenyl)benzamide using benzamide other than aniline. More concretely, there is described preparing 4-nitroaniline by synthesizing N-(4-nitrophenyl)benzamide using nitrobenzene in the amount of about 1 mole based on benzamide and then by hydrolyzing the resulting product with water or ammonia. However, there is no mention of the preparation of 4,4'-nitrodiphenylamine. This seems to be because N-(4-nitrophenyl)benzamide produced by a reaction of amide and nitrobenzene is a stable and separable compound, so that it is no longer reacted with nitrobenzene.

Similar reactions are disclosed in the following documents of the prior art.

WO 94/08953 discloses a process for preparing an isocyanate having at least two -N=C=O groups and a process for preparing a carbamate ester having at least two -NH-COOR groups. These processes comprise preparing a substituted aromatic amine having at least two -NH₂ groups. The substituted aromatic amine having at least two -NH₂ groups can be prepared by different processes including contacting an amide which also may be urea and nitrobenzene in the presence of a suitable base and reducing the reaction products to produce a substituted aromatic amine having at least two -NH₂ groups.

Iida et al discloses in Nippon Kagaku Kaishi (1976), 1, 138-43, with the title "Studies on the base-catalyzed reaction. VII. The reactions of p-nitroaniline with nitrobenzene derivatives in strongly basic solutions" reactions according to the title.

JP 63316762 discloses a new class of compounds which are produced by reacting cyanamides with a substituted diaminodiphenylamine derivative. One example of such a compound is 4,4'-[N-(Cyclohexyl)imino]-bis(phenylguanindine) dihydrochloride.

US 4187248 discloses a process for producing nitrodiarylamines. These molecules are achieved by reacting an alkali metal salt of a formamide with a nitrohaloarene.

### Disclosure of the Invention

The present invention employs the NASH reaction as mentioned above, and utilizes urea instead of aniline or benzamide. More particularly, the present invention is a method of selectively preparing 4,4'-dinitrodiphenylamine by reacting urea with an excess of nitrobenzene. 4-Nitroaniline, which is an intermediate produced with the present invention, is increased in production at the beginning of the reaction, and easily reacted with nitrobenzene to produce 4,4'-dinitrodiphenylamine. The method in accordance with the present invention is advantageous in that the reaction is relatively simple while allowing a selective production of only 4,4'-dinitrodiphenylamine using a relatively cheap alkali base without producing other by-products.

We have made diligent efforts to find an improved method for preparing 4,4'-dinitrodiphenylamine, which does not involve production of by-products and requirement of any post-treating process. As a result, we have found that 4,4'-dinitrodiphenylamine can be produced without production of by-products and requirement of any post-treating process while reducing the manufacturing costs when urea, which is relatively cheap, is directly reacted with nitrobenzene in a polar organic solvent using a relatively cheap base. It has also been found that the production of 4,4'-dinitrodiphenylamine can be achieved in high yield and selectivity even under non-intensive reaction conditions by appropriately controlling the amounts of urea and nitrobenzene. Based on such facts, the present invention has been achieved.

In accordance with the present invention, an inorganic or organic base may be used for the base existing in the polar organic solvent for the reaction of urea and nitrobenzene to achieve a selective production of 4,4'-dinitrodiphenylamine. Preferably, an alkali metal or alkaline earth metal base, such as sodium hydroxide, may be used for the inorganic base. On the other hand, a base such as TMA (OH) may be preferably used for the organic base. In the reaction of urea and nitrobenzene in accordance with the present invention, 4-nitroaniline is produced at the beginning of the reaction. Using an extended period of reaction time, however, such 4-nitroaniline is reacted with nitrobenzene, thereby producing 4,4'-dinitrodiphenylamine. Consequently, only 4,4'-dinitrodiphenylamine can be obtained without any by-products. 4-Nitroaniline is produced in a fast rate at the beginning of the reaction while reacting with nitrobenzene as the reaction further proceeds, thereby producing only 4,4'-dinitrodiphenylamine. On the other hand, 4,4'-dinitrodiphenylamine is slowly produced at the beginning of the reaction while being produced at an increased rate as the reaction of 4-nitroaniline and nitrobenzene proceeds. The reaction is conducted in such a fashion that it is completed at the point of time when 4-nitroaniline is completely consumed. Moreover, the reaction is carried out under an oxygen atmosphere to prevent azoxybenzene from being produced.

### Best Mode for Carrying Out the Invention

The foregoing and other objects, features and advantages of the invention will be apparent to those skilled in the art to which the present invention relates from reading the following specification.

The present invention is directed to a method of selectively preparing 4,4'-dinitrodiphenylamine by reacting urea and nitrobenzene in a polar organic solvent in the presence of an organic or inorganic base.

In the practice of the present invention, dimethylsulfoxide (hereinafter, called "DMSO"), which is a polar organic solvent, is preferably used in view of solubilities of urea and nitrobenzene.

The molar ratio of nitrobenzene to urea used is in the range of about 1:1 to about 16:1, with the preferred ratio in view of yield being in the range of about 4:1 to about 8:1. The volume ratio of the solvent to urea and nitrobenzene used is in the range of 50:1 to 1:1, with the preferred ratio being in the range of 30:1 to 1:1.

Examples of the base used in accordance with the present invention include, but are not limited to, organic and inorganic bases, such as sodium hydroxide (NaOH), potassium hydroxide (KOH), potassium t-butoxide (t-BuOK), sodium hydride(NaH), and TMA(OH). The molar ratio of the base to urea used is in the range of about 1:1 to about 20:1. The higher the amount of the base used, the higher the yield. However, where the molar ratio of the base to urea is 12:1 or above, the yield is rather decreased. On the other hand, where the molar ratio of the base to urea is 4:1 or below, it shows a result that 4-nitroaniline remains even when the reaction is carried out at a temperature of 60 °C for 8 hours.

The reaction temperature is in the range of room temperature to 100°C, with the preferred reaction temperature being in the range of 50 to 80 °C. Where the reaction temperature is low, such as less than 50 °C, the reaction rate is too slow. On the other hand, where the reaction temperature exceeds 80 °C, the yield is remarkably decreased due to the decomposition of urea. The lower the reaction temperature, the longer the time during which 4-nitroaniline produced at the beginning of the reaction is converted into 4,4'-dinitrodiphenylamine. Of course, the extended reaction time results in an increase in yield of a product. On the other hand, at the reaction temperature exceeding 80 °C, the yield of the product is high at the beginning of the reaction. However, the yield of the product increases no longer from the level at the beginning of the reaction due to a decomposition of urea. Accordingly, the reaction temperature is determined to be ranged from 50 °C to 80 °C in order to prevent a decomposition of urea while increasing the reaction rate, thereby obtaining an increased yield of the product.

Moreover, we surprisingly found that, where the moisture content in the reaction system is 5% or less based on the weight of the entire reaction solution, it does not have a significant effect on a reactivity. Thus, no particular process to remove the moisture of the solvent is used. Where the reaction is carried out in a state where water is added to the solvent in an amount of 1%, a high yield is exhibited at the beginning of the reaction. In this case, however, the yield exhibited after the reaction of about 6 hours is not so high, as compared to that of the case where water is not added. In addition, an excess of azoxybenzene is produced under an atmosphere not containing oxygen whereas no azoxybenzene is produced under an atmosphere containing oxygen.

### Examples

The following examples are for illustration purposes only and in no way limit the scope of this invention.

In the following examples, products were analyzed by the Nuclear Magnetic Resonance(NMR) spectrum and the Gas Chromatography-Mass Spectroscopy Detector to identify them. Also, the products were quantitatively analyzed according to the following condition by high performance liquid chromatography. Such a high performance liquid chromatography was carried out using a product manufactured by Hitachi Co. in Japan and consisting of an L-6200 intelligent pump and an L-4200 UV-VIS detector. In high performance liquid chromatography, all quantitative values were measured at a wavelength of 254 nm, and developing speed was 1 ml/minute. Furthermore, in high performance liquid chromatography, a Cosmosil 5C18-AR ( 4.6 x150 mm) packed column was used. An elution gradient of high performance liquid chromatography was as follows.

| Elution gradient | | |
|---|---|---|
| | Solvent A | Solvent B |
| Time (minute) | % Distilled water | % Acetonitrile |
| 0 | 65 | 35 |
| 25 | 0 | 100 |
| 33 | 65 | 35 |

For the quantitative analysis of each product, pyrene was used as an internal standard. An area ratio for a concentration of each material was calculated relative to an area of pyrene and standard-calibrated. A molar concentration of a product was calculated from the calibration curve.

### Example 1

Into a 100ml three-necked reactor equipped with a condenser and a thermometer were charged 1.2 g (20 mol.) of urea, 9.6 g (240 mol.) of sodium hydroxide, 0.1 g of pyrene, and 30 ml of DMSO solvent. Then, the resulting mixture was added with 8.2 ml (80 mol.) of nitrobenzene while stirring. Thereafter the resulting solution was stirred for 30 minutes while passing oxygen, so that it was subsequently allowed to conduct a reaction at a temperature of 60 °C. A progressed degree of the reaction was confirmed by HPLC. After the reaction, the product was cooled. Analysis of the product by HPLC showed that, where the reaction was carried out for 6 hours, 4,4'-dinitrodiphenylamine was obtained in a yield of 82 mol%, and where the reaction was carried out for 8 hours, 4,4'-dinitrodiphenylamine was obtained in a yield of 94 mol%.

### Example 2

Reactions were carried out under the same conditions as those in Example 1 except that the reaction temperature for each reaction was changed. Results are shown in Table 1 below.

**Table 1: Change in yield with change in reaction temperature**

| Reaction temperature (°C) | Time (hr) | Yield (mol %) | |
|---|---|---|---|
| | | 4 - NA^{A} | DNDPA^{B} |
| 80 | 8 | - | 59 |
| 70 | 6 | - | 67 |
| 6C | 6 | - | 82 |
| | 8 | - | 94 |
| 50 | 6 | 8.9 | 55 |
| | 8 | 6.8 | 63 |

| | | | |
|---|---|---|---|
| A) 4-NA : 4-nitroaniline | | | |
| B) DNDPA: 4,4'-dinitrodiphenylamine | | | |

### Example 3

Reactions were carried out under the same conditions as those in Example 1 except that the kind of base and the amount of the base for each reaction were changed. Results are shown in Table 2 below.

**Table 2: Change in yield with change in the kind of base**

| Base | Base amount | Time (hr) | Yield (mol%) | |
|---|---|---|---|---|
| | (g(mol.)) | | 4-NA^{A} | DNDPA^{B} |
| Sodium hydroxide | 9.6 (240) | 8 | - | 94 |
| Potassium hydroxide | 17.9 (320) | 8 | 9 | 52 |
| Potassium t-butoxide^{C} | 4.5 (40) | 8 | - | 37 |
| TMA(OH)^{D} | 8.6 (47) | 8 | - | 51 |

| | | | | |
|---|---|---|---|---|
| A) 4-NA : 4-nitroaniline | | | | |
| B) DNDPA: 4,4'-dinitrodiphenylamine | | | | |
| C) and D) : 0.6 g (10 mol.) of urea was used. | | | | |

### Example 4

Reactions were carried out under the same conditions as those in Example 1 except that the amount of the base for each reaction was changed. Results are shown in Table 3 below.

**Table 3: Change in yield with change in the amount of base**

| Base amount | Reaction time | Yield (%) | |
|---|---|---|---|
| (g(mol.)) | (hr) | 4-NA^{A} | DNDPA^{B} |
| 3.2 (80) | 8 | 14 | 55 |
| 6.4 (160) | 8 | 5 | 67 |
| 9.6 (240) | 8 | - | 94 |
| 12.8 (320) | 8 | - | 92 |

| | | | |
|---|---|---|---|
| A) 4-NA : 4-nitroaniline | | | |
| B) DNDPA: 4,4'-dinitrodiphenylamine | | | |

### Example 5

Reactions were carried out under the same conditions as those in Example 1 except that the amount of DMSO solvent for each reaction was changed. Results are shown in Table 4 below.

**Table 4: Change in yield with change in the amount of solvent**

| Amount of DMSO | Reaction time | Yield (%) | |
|---|---|---|---|
| (ml) | (hr) | 4-NA^{A} | DNDPA^{B} |
| 20 | 8 | - | 65 |
| 30 | 8 | - | 94 |
| 40 | 8 | 4 | 72 |

| | | | |
|---|---|---|---|
| A) 4-NA : 4-nitroaniline | | | |
| B) DNDPA: 4,4'-dinitrodiphenylamine | | | |

### Example 6

Reactions were carried out under the same conditions as those in Example 1 except that the amount of nitrobenzene to the amount of urea for each reaction was changed. Results are shown in Table 5 below.

**Table 5: Change in yield with change in the amount of nitrobenzene**

| Amount of | Reaction time | Yield (%) | |
|---|---|---|---|
| nitrobenzene (ml (mol.)) | (hr) | 4-NA^{A} | DNDPA^{B} |
| 4.1 (40) | 8 | 7 | 67 |
| 8.2 (80) | 8 | - | 94 |
| 16.4 (160) | 8 | - | 75 |

| | | | |
|---|---|---|---|
| A) 4-NA : 4-nitroaniline | | | |
| B) DNDPA: 4,4'-dinitrodiphenylamine | | | |

### Example 7

Reactions were carried out under the same conditions as those in Example 1 except that moisture content for each reaction were changed. Results are shown in Table 6 below.

**Table 6: Change in yield with change in moisture content**

| Drying agent and | Reaction time | Yield (%) | |
|---|---|---|---|
| moisture content | (hr) | 4-NA^{A} | DNDPA^{B} |
| None^{C} | 8 | - | 94 |
| K₂CO₃^{D} | 8 | - | 73 |
| 1% Water^{E} | 8 | - | 70 |
| 5% Water^{F} | 8 | 3 | 58 |

| | | | |
|---|---|---|---|
| A) 4-NA : 4-nitroaniline | | | |
| B) DNDPA: 4,4'-dinirrodiphenylamine | | | |
| C): Commercially available solvent was used without further purification (no drying agent was used). | | | |
| D): A drying agent was used to remove moisture in a reaction system. | | | |
| E) : Distilled water was added in the amount of 1% based on the volume of the solvent. | | | |
| F): Distilled water was added in the amount of 5% based on the volume of the solvent. | | | |

### Example 8

Reactions were carried out under the same conditions as those in Example 1 except that an atmosphere for each reaction was changed. Results are shown in Table 8 below.

**Table 8: Change in yield with reaction atmosphere**

| Reaction | Reaction time | Yield (%) | |
|---|---|---|---|
| atmosphere | (hr) | 4-NA^{A} | DNDPA^{B} |
| Oxygen | 8 | - | 94 |
| Nitrogen | 8 | 2 | 50 |
| Air | 8 | 1 | 75 |
| General atmosphere | 8 | 3 | 56 |

| | | | |
|---|---|---|---|
| A) 4-NA : 4-nitroaniline | | | |
| B) DNDPA: 4,4'-dinitrodiphenylamine | | | |

### Industrial Applicability

As apparent from the above description, the method of the present invention for preparing 4,4'-dinitrodiphenylamine uses relatively cheap urea and nitrobenzene, as raw materials, while using a relatively cheap base, thereby decreasing the manufacturing costs. Moreover, the method of the present invention does not produce by-products, thus eliminating a need for a post-treating process. Additionally, the method of the present invention is advantageous in that the production of 4,4'-dinitrodiphenylamine can be achieved in high yield and selectivity even under non-intensive reaction conditions by appropriately controlling the amounts of urea and nitrobenzene.

## Claims

1. A method for preparing 4,4'-dinitrodiphenylamine, comprising reacting urea and nitrobenzene in a polar organic solvent in the presence of a base at a temperature of room temperature to 100 °C.

2. The method of claim 1, wherein the molar ratio of the nitrobenzene to the urea is in the range of 2:1 to 10:1.

3. The method of claim 1, wherein the molar ratio of the base to the urea is in the range of 1:1 to 20:1.

4. The method of claim 1, wherein the volume ratio of the polar organic solvent to the reactants is in the range of 1:1 to 50:1.

5. The method of claim 1, wherein the base is selected from the group consisting of sodium hydroxide, sodium hydride, potassium hydroxide, potassium t-butoxide, and tetramethylammonium hydroxide.

6. The method of claim 1, wherein the polar organic solvent is dimethylsulfoxide (DMSO).

7. The method of claim 1, wherein the reaction is carried out in a nitrogen, oxygen, or air atmosphere.

## Patentansprüche

1. Eine Methode zur Herstellung von 4,4'-Dinitrodiphenylamin, einschließlich reagierender Harnstoff und Nitrobenzen in einem polaren, organischen Lösungsmittel in Anwesenheit einer Base bei einer Temperatur von Raumtemperatur bis 100°C.

2. Die Methode aus Anspruch 1, wobei das molare Verhältnis von Nitrobenzen zu Harnstoff im Bereich von 2:1 bis 10:1 liegt.

3. Die Methode aus Anspruch 1, wobei das molare Verhältnis von Base zu Harnstoff im Bereich von 1:1 bis 20:1 liegt.

4. Die Methode aus Anspruch 1, wobei das Volumenverhältnis des polaren, organischen Lösungsmittels zu den Reaktanten im Bereich von 1:1 bis 50:1 liegt.

5. Die Methode aus Anspruch 1, wobei die Base aus der Gruppe bestehend aus Natriumhydroxid, Natriumhydrid, Kaliumhydroxid, Kalium-t-butoxid und Tetramethylammoniumhydroxid ausgewählt wird.

6. Die Methode aus Anspruch 1, wobei das polare, organische Lösungsmittel Dimethylsulfoxid (DMSO) ist.

7. Die Methode aus Anspruch 1, wobei die Reaktion in einer Stickstoff-, Sauerstoff- oder Luftatmosphäre durchgeführt wird.

## Revendications

1. Procédé de préparation de la 4,4'-dinitrodiphénylamine, comprenant l'étape consistant à faire réagir de l'urée et du nitrobenzène dans un solvant organique polaire en présence d'une base à une température comprise entre la température ambiante et 100°C.

2. Procédé selon la revendication 1, dans lequel le rapport molaire entre le nitrobenzène et l'urée est dans la gamme allant de 2:1 à 10:1.

3. Procédé selon la revendication 1, dans lequel le rapport molaire entre la base et l'urée est dans la gamme allant de 1:1 à 20:1.

4. Procédé selon la revendication 1, dans lequel le rapport en volume ente le solvant organique polaire et les réactifs est dans la gamme allant de 1:1 à 50:1.

5. Procédé selon la revendication 1, dans lequel la base est choisie dans le groupe consistant en l'hydroxyde de sodium, l'hydrure de sodium, l'hydroxyde de potassium, le t-butoxyde de potassium, et l'hydroxyde de tétraméthylammonium.

6. Procédé selon la revendication 1, dans lequel le solvant organique polaire est le diméthylsulfoxyde (DMSO).

7. Procédé selon la revendication 1, dans lequel la réaction est effectuée dans une atmosphère d'azote, d'oxygène, ou d'air.
